# EUROPÄISCHE PATENTANMELDUNG

(11) **EP 0 702 956 A1**
(43) Veröffentlichungstag der Anmeldung: **27.03.1996**
(21) Anmeldenummer: 95113082.2
(22) Anmeldetag: 19.08.1995
(51) Int. Cl.: A61K 35/78, A61K 7/48

(54) **Mittel zur Behandlung oder Pflege gereizter oder entzündeter Haut, enthaltend einen Extrakt aus getrocknetem oder frischem Spitzwegerichkraut (Plantago lanceolata L.) in einer Grundlage, die die Anwendung auf der Haut als Stift, Roller oder Creme ermöglicht**

(30) Priorität: 21.09.1994 DE 4433666
(71) Anmelder: ASTA Medica Aktiengesellschaft, D-01277 Dresden (DE)
(72) Erfinder: Hose, Sebastian, Dr., D-60327 Frankfurt (DE); Carle, Reinhold, Dr., D-63322 Rödermark (DE); Werner, Ulrich, Dr., D-56357 Miehlen (DE)

(57) **Zusammenfassung**

Trockenextrakte aus Spitzwegerichkraut (Plantago lanceolata, L.), erhalten durch Fluidextraktion, werden in Mitteln zur Behandlung von Insektensticken und ähnlichem verwendet.
Die erfindungsgemäße Zubereitung eignet sich in die Einarbeitung in Roller, Öle, Lotionen und Stifte.

## Beschreibung

Tees und Extrakte aus Spitzwegerichkraut (Plantago lanceolata L., Plantaginaceae) werden in der Volksmedizin zur Reizlinderung bei Katarrhen der oberen Luftwege verwendet. Entzündungen des Mund- und Rachenraumes werden mit Mazeraten, Fluidextrakten, Sirup und dem Preßsaft der frischen Pflanze sowie Pastillen behandelt. Zubereitungen aus den Samen von Plantago-Arten finden in ähnlicher Weise Anwendung.

Die Monographie der BGA-Kommission E (BAnz Nr. 223 vom 30.11.85) für Plantaginis lanceolatae herba (Spitzwegerichkraut) nennt entzündliche Veränderungen der Haut als Anwendungsgebiet für die frischen oder getrockneten oberirdischen Teile der Pflanze. Verwendet werden die zerkleinerte Droge sowie andere galenische Zubereitungen, wie beispielsweise Extrakte, Sirupe, Preßsäfte, Tabletten und Dragees.

In der Volksmedizin wird der Preßsaft des frischen Krautes äußerlich als wundheilendes und entzündungshemmendes Mittel eingesetzt.

Nach Hager Handbuch der pharmazeutischen Praxis 4. Auflage Springer Verlag(1977), Seite 747 ff. besitzen Extrakte von Plantago lanceolata antibakterielle Eigenschaften. Die größte Wirkung haben junge Blätter gegen Staphylococcus aureus, Streptococcus betahämolyticus und Bacillus subtilis. Als Hauptwirkstoff wird Aucubigenin angesehen.

Nach R.F. Lehrbuch der Phytotherapie Hippokrates Verlag Stuttgart Weiß (1990), Seite 262 ff. werden Blätter des Breitwegerichs und des Spitzwegerichs im frischen Zustand bei Insektenstichen verwendet.

Das ungarische Patent 63324 2A beschreibt eine Zubereitung (Lotion), die Wegerich-Extrakte in Kombination mit weiteren Pflanzenextrakten (zum Beispiel Schafgarbe) und Vitamin B 12 enthält. Das Präparat dient der Behandlung von Akne vulgaris.

Das ungarische Patent 833 67 beschreibt pharmazeutische und kosmetische Präparate, die Inhaltsstoffe der Samen des Breitwegerichs (Plantago major L.) enthalten. Diese Zubereitungen können für eine Vielzahl von Erkrankungen angewendet werden, insbesondere als Spülungen und Tees gegen Entzündungen. Anwendungen als Kosmetika sind ebenfalls vorgesehen.

Das ungarische Patent 2077 92 beschreibt eine Zahnpasta, die einen Extrakt aus Plantago lanceolata und Solidago canadensis (Goldrute) enthält. Die Zahnpasta dient der vorbeugenden Behandlung von Entzündungen der Mundschleimhaut und des Zahnfleisches.

Die rumänische Patentschrift 60 244 betrifft ein wundheilungsförderndes, antiseptisches und entzündungshemmendes Produkt, das Spitzwegerich-Extrakt enthält und in Form eines Aerosols äußerlich angewendet wird.

Entzündungshemmende Eigenschaften und die Wirksamkeit bei Insektenstichen werden mit frischen Blättern von Plantago lanceolata und entsprechenden Zubereitungen beobachtet.

Überraschenderweise zeigte nun jedoch die topische Applikation eines alkoholischen Extraktes aus getrocknetem Spitzwegerich-Kraut (Fluidextrakt n. Erg. Bd. 6; DEV 1:1-2) eine antientzündliche Wirkung im Phospholipase A (aus Bienengift) induzierten Ödem am narkotisierten Hausschwein. Als Extraktionsmittel können Alkohole, wie beispielsweise Ethanol und Isopropanol, Propylenglykole, Methanol verwendet werden, weiter können Ketone eingesetzt werden, wie beispielsweise Aceton, Methylethylketon; weiter Ester, wie beispielsweise Essigsäureethylester.
Auch Mischungen aus den vorstehend genannten Extraktionsmitteln untereinander und mit Wasser können eingesetzt werden.
Als weiteres Extraktionsmittel kann auch überkritisches Kohlendioxid eingesetzt werden.
Man erhält durch Extraktion einen Flüssigextrakt, der als solcher weiterverarbeitet werden kann oder nach dem Verdampfen des Lösungsmittels einen Trockenextrakt, der ebenfalls weiter verarbeitet werden kann.
Dieser Effekt war nicht vorhersehbar und ist umso überraschender, da im allgemeinen bekannt ist, daß durch Trocknungsvorgänge von Pflanzenmaterial Wirkstoffverluste beziehungsweise -veränderungen einhergehen. Der Extrakt hemmte die Ausdehnung des entzündlichen Ödems signifikant um 42 %. Da eine 77%ige wäßrigalkoholische Lösung alleine bereits eine Hemmung um 22 % bewirkte, resultiert hieraus eine Substanzwirkung von ca. 20 %. In einem weiteren Versuch wurde der Extrakt in eine Creme- bzw. Emulsionsgrundlage eingearbeitet. Diese Zubereitungen inhibierten die Formierung der entzündlichen Hautquaddeln in Abhängigkeit von der Wirkstoffkonzentration. Die 5-, 10- und 20 %ige Spitzwegerich-Creme/Emulsion zeigten signifikante Ödemhemmung um 42, 50 beziehungsweise 59 %. Die Cremegrundlage ohne Wirkstoff zeigte keinerlei Beeinflussung des PLA induzierten Hautödems. Aus diesen Versuchen ergibt sich, daß die ED₅₀ bei einer 10%igen Creme/Emulsion liegt.

Zur Behandlung juckender, allergischer und entzündlicher Hauterkrankungen werden topisch applizierbare Zubereitungen von Histamin-Antagonisten (Antihistaminika) angewendet. Hierzu zählen Verbindungen wie zum Beispiel Bamipinlactat, Clemastinhydrogenfumarat, Diphenhydramin-HCl, Tripelenamin-HCl und Chlorphenoxamin.

Überraschenderweise zeigte eine handelsübliche, 1,5 % Chlorphenoxamin enthaltende Creme im PLA induzierten Hautödem eine deutlich schwächer ausgeprägte antiödematöse Wirkung (25 % Ödemhemmung) als eine entsprechende Zubereitung (Creme/Emulsion), die 10 % Spitzwegerich-Fluidextrakt enthält (50 % Ödemhemmung).

Handelsübliche Anwendungsformen sind neben Cremes, Gelen, Salben, Lotionen auch Stifte, die Antihistaminika bzw. andere Stoffe zur Behandlung oder Pflege der entzündeten oder gereizten Haut enthalten. Hierzu zählen Präparate die z.B. Tripelennamin-HCl (Azaron® Stift), Menthol oder Allantoin (Soventol® Stift) oder Ammoniumhydroxid (After-Bite® Stift) enthalten.

Extrakte von frischen oder getrockneten Pflanzenteilen von Plantago lanceolata lassen sich in Konzentrationen von 1 bis 30 % in halbfeste Darreichungsformen einarbeiten (Creme, Emulsion, Stift), die die Anwendung des Extraktes auf der Haut ermöglichen.

Als Grundlagen für die Anwendungsformen können alle in den üblichen Handbüchern beschriebenen Rezepturen und Hilfsstoffe verwendet werden, wie beispielsweise in List, "Arzneiformenlehre", Stuttgart 1985; Voigt, "Lehrbuch der pharmazeutischen Technologie", Weinheim, 1987 und "Pharmazeutische Technologie" (Herausgeber: Sucker, Fuchs, Speiser), Stuttgart 1991.

Das erfindungsgemäße Mittel enthält Extrakte aus frischen oder getrockneten oberirdischen Pflanzenteilen von Plantago lanceolata L. in Konzentrationen von 1 bis 30 %, vorzugsweise 5 bis 20 % und insbesondere 8 bis 12 %, gegebenenfalls mit einem Zusatz von 0,01 - 0,1 % Menthol. Es ist als Lotion, Creme oder Fettgrundlage in Form von Stiften oder Rollern auf der Haut anwendbar.

### Beispiel 1

| Spitzwegerichcreme | |
|---|---|
| Glycerolmonostearat | 4,0 |
| Cetylalkohol | 6,0 |
| Polyoxyäthylenglycerolmonostearat | 7,0 |
| Miglyol 812 | 7,5 |
| Propylenglykol | 10,0 |
| Wasser | 55,5 |
| Spitzwegerichextrakt | 10,0 |
| Propyl-4-hydroxybenzoat | 0,03 |
| Methyl-4-hydroxybenzoat | 0,07 |

### Beispiel 2

| Spitzwegerichcreme | |
|---|---|
| Glycerolmonostearat | 1,0 |
| Cetylalkohol | 4,0 |
| Polyoxyäthylenglycerolmonostearat | 5,0 |
| Miglyol 812 | 13,0 |
| Paraffin, dickflüssig | 2,0 |
| Wasser | 65,0 |
| Spitzwegerichextrakt | 10,0 |
| Propyl-4-hydroxybenzoat | 0,03 |
| Methyl-4-hydroxybenzoat | 0,07 |

### Beispiel 3

| Spitzwegerichgel | |
|---|---|
| Hydroxypropylcellulose | 1,0 |
| Wasser | 54,0 |
| Ethanol 96% | 30,0 |
| Propylenglykol | 5,0 |
| Spitzwegerichextrakt | 10,0 |

### Beispiel 4

| Spitzwegerichgel | |
|---|---|
| Hydroxypropylcellulose | 2,0 |
| Wasser | 53,0 |
| Ethanol 96% | 30,0 |
| Propylenglykol | 5,0 |
| Spitzwegerichextrakt | 10,0 |

### Beispiel 5

| Spitzwegerichgel | |
|---|---|
| Hydroxypropylcellulose | 3,0 |
| Wasser | 52,0 |
| Ethanol 96% | 30,0 |
| Propylenglykol | 5,0 |
| Spitzwegerichextrakt | 10,0 |

### Beispiel 6

| Spitzwegerichlösung | |
|---|---|
| Wasser | 55,0 |
| Ethanol 96% | 30,0 |
| Propylenglykol | 5,0 |
| Spitzwegerichextrakt | 10,0 |

### Beispiel 7

| Spitzwegerichemulsion | |
|---|---|
| Glycerylstearat | 8,0 |
| Ceteareth-12 | 3,0 |
| Miglyol 812 | 5,0 |
| Octyl Dodecanol | 3,0 |
| Wasser | 70,5 |
| Spitzwegerichextrakt | 10,0 |
| Phenoxyethanol | 0,5 |

### Beispiel 8

| Spitzwegerichemulsion | |
|---|---|
| Glycerylstearat | 5,0 |
| Ceteareth-12 | 4,5 |
| Ceteareth-20 | 1,5 |
| Miglyol 812 | 10,0 |
| Wasser | 68,5 |
| Spitzwegerichextrakt | 10,0 |
| Phenoxyethanol | 0,5 |

### Beispiel 9

| Spitzwegerichstift | |
|---|---|
| Stearinsäuremonoethanolamid | 25,0 |
| Wachs, gebleicht | 23,0 |
| Octyl Dodecanol | 30,0 |
| Cetylalkohol | 12,0 |
| Ethanol 96% | 20,0 |
| Spitzwegerichextrakt | 10,0 |
| Propyl-4-hydroxybenzoat | 0,03 |
| Methyl-4-hydroxybenzoat | 0,07 |

### Beispiel 10

| Spitzwegerichgel mit 0,05% Menthol | |
|---|---|
| Hydroxypropylcellulose | 25,0 |
| Wasser | 52,95 |
| Ethanol 96% | 30,0 |
| Propylenglykol | 5,0 |
| Spitzwegerichextrakt | 10,0 |

## Patentansprüche

1. Mittel zur Behandlung oder Pflege der entzündeten oder gereizten Haut, dadurch gekennzeichnet, daß es als wirksame Komponente Extrakte von Plantago lanceolata L. in einer Konzentration von 1 bis 30 Gew.% enthält.

2. Mittel zur Behandlung oder Pflege der entzündeten oder gereizten Haut, dadurch gekennzeichnet, daß es als wirksame Komponente Extrakte von Plantago lanceolata L. in einer Konzentration von 5 bis 20 Gew.% enthält.

3. Mittel zur Behandlung oder Pflege der entzündeten oder gereizten Haut, dadurch gekennzeichnet, daß es als wirksame Komponente Extrakte von Plantago lanceolata L. in einer Konzentration von 8 bis 12 Gew.% enthält.

4. Mittel gemäß Ansprüchen 1 bis 3, dadurch gekennzeichnet, daß der Spitzwegerichextrakt aus den frischen oder getrockneten Teilen von Plantago lanceolata gewonnen wird.

5. Mittel gemäß Anspruch 4, dadurch gekennzeichnet, daß als Extraktionsmittel Alkohole oder Ketone, Ester oder deren Mischungen mit Wasser verwendet werden.

6. Mittel nach vorstehenden Ansprüchen, dadurch gekennzeichnet, daß der Extrakt in trockener oder flüssiger Form verwendet wird.

7. Verwendung des Extraktes nach vorstehenden Ansprüchen in Form eines Öls, einer Lotion, einer Creme oder eines Gels.

8. Verwendung der Extrakte nach einem der vorstehenden Ansprüche als Stift.

9. Verwendung der Extrakte nach einem der vorstehenden Ansprüche als Roller.

10. Verwendung des Mittels, gemäß Anspruch 1 zur Herstellung eines Mittels zur Behandlung der gereizten Haut.

11. Verwendung des Mittels, gemäß Anspruch 1 zur Pflege der gereizten Haut.

12. Verwendung des Mittels, gemäß Anspruch 2 zur Behandlung der gereizten Haut.

13. Verwendung des Mittels, gemäß Anspruch 2 zur Pflege der gereizten Haut.

14. Verwendung des Mittel, gemäß Anspruch 3 zur Behandlung der gereizten Haut.

15. Verwendung des Mittels, gemäß Anspruch 3 zur Pflege der gereizten Haut.
